# EUROPEAN PATENT APPLICATION

(11) **EP 4 545 510 A1**
(43) Date of publication of application: **30.04.2025**
(21) Application number: 24780922.1
(22) Date of filing: 29.03.2024
(51) Int. Cl.: C07C 17/386, C07C 19/08

(54) **METHOD FOR PRODUCING PURIFIED 1,1,2-TRIFLUOROETHANE (HFC-143), AND COMPOSITION CONTAINING HFC-143**

(30) Priority: 31.03.2023 JP 2023059376
(71) Applicant: DAIKIN INDUSTRIES, LTD., Osaka-shi, Osaka 530-0001 (JP)
(72) Inventor: TAKAHASHI, Kazuhiro, Osaka-Shi, Osaka 530-0001 (JP); ISHIHARA, Kaito, Osaka-Shi, Osaka 530-0001 (JP); TABUCHI, Akikazu, Osaka-Shi, Osaka 530-0001 (JP); NOGUCHI, Atsushi, Osaka-Shi, Osaka 530-0001 (JP); NAKAO, Yuki, Osaka-Shi, Osaka 530-0001 (JP)
(74) Representative: Hoffmann Eitle
(86) International application number: PCT/JP2024/013357
(87) International publication number: WO 2024/204829

(57) **Abstract**

An object of the present disclosure is to provide a method for efficiently purifying HFC-143, and a composition comprising HFC-143.

A method for producing purified HFC-143.

## Description

### Technical Field

The present disclosure relates to a method for producing purified 1,1,2-trifluoroethane (HFC-143) and a composition comprising HFC-143.

### Background Art

HFC-143 is useful not only as an intermediate for obtaining HFO-1132, but also itself as a refrigerant (Patent Literature 1).

### Citation List

### Patent Literature

PTL 1: WO1994/11460

### Summary of Invention

### Technical Problem

An object of the present disclosure is to provide a method for efficiently purifying HFC-143 and a composition comprising HFC-143.

### Solution to Problem

The present disclosure includes, for example, the subject matter described in the following items.
Item 1. A method for producing purified 1,1,2-trifluoroethane (HFC-143), comprising (1) an extractive distillation step of bringing a composition comprising 1-chloro-1,1,2-trifluoroethane (HCFC-133b) and/or 1,2-dichloro-1,1,2-trifluoroethane (HCFC-123a), and HFC-143 into contact with a solvent A to obtain a composition having a reduced amount of HCFC-133b and/or HCFC-123a compared with the original composition,
   the solvent A being at least one member selected from the group consisting of an amine, a halogen compound, and toluene, and
   the halogen compound being at least one member selected from the group consisting of chloroform, hexafluorobenzene, 1-chlorobutane, 1,3-bis(trifluoromethyl)benzene, monofluorobenzene, and α,α,α-trifluorotoluene.
Item 2. The method according to Item 1, further comprising (2) a distillation step of subjecting a composition comprising HCFC-133b and/or HCFC-123a, and the solvent A obtained from the bottom of an extractive distillation column through the extractive distillation step (1) to distillation to separate the composition into HCFC-133b and/or HCFC-123a, and the solvent A.
Item 3. The method according to Item 1 or 2, wherein the extractive distillation step (1) comprises extractive distillation performed under a pressure of 0.05 to 5 MPaG (gauge pressure) using a first distillation column.
Item 4. The method according to Item 2 or 3, wherein the distillation step (2) comprises distillation performed under a pressure of 0.05 to 3 MPaG (gauge pressure) using a second distillation column.
Item 5. The method according to any one of Items 1 to 4, further comprising (3) a solvent recovery step of recovering the solvent A used in the extractive distillation step (1), and recirculating the recovered solvent A to the extractive distillation step (1).
Item 6. The method according to any one of Items 1 to 5, wherein the amine is represented by formula: NR¹R²R³ wherein R¹, R², and R³ are the same or different, and each represents hydrogen or an optionally substituted C₁₋₃ hydrocarbon group, with the proviso that a case in which all of R¹, R², and R³ are hydrogen is excluded.
Item 7. The method according to any one of Items 1 to 6, wherein the amine has a standard boiling point of -10 to 160°C.
Item 8. The method according to any one of Items 1 to 7, wherein the amine is at least one member selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine.
Item 9. The method according to any one of Items 1 to 7, wherein the amine is at least one member selected from the group consisting of diethylamine and triethylamine.
Item 10. The method according to any one of Items 1 to 9, wherein the halogen compound is at least one member selected from the group consisting of chloroform, hexafluorobenzene, 1-chlorobutane, 1,3-bis(trifluoromethyl)benzene, and monofluorobenzene.
Item 11. (Refrigerant 1) A composition comprising 1,1,2-trifluoroethane (HFC-143), 1-chloro-1,1,2-trifluoroethane (HCFC-133b) and/or 1,2-dichloro-1,1,2-trifluoroethane (HCFC-123a), and a substance A,
   the total concentration of the three components being 99.5 mass% or more based on the entire composition,
   the content of HCFC-133b and/or HCFC-123a being more than 0 mass% and 0.4 mass% or less based on the entire composition,
   the content of the substance A being more than 0 mass% and 0.1 mass% or less based on the entire composition,
   the substance A being at least one member selected from the group consisting of an amine, a halogen compound, and toluene, and
   the halogen compound being at least one member selected from the group consisting of chloroform, hexafluorobenzene, 1-chlorobutane, 1,3-bis(trifluoromethyl)benzene, monofluorobenzene, and α,α,α-trifluorotoluene.
Item 12. (Composition 1) A composition comprising 1,1,2-trifluoroethane (HFC-143) and a substance A,
   the total concentration of the two components being 99.5 mass% or more based on the entire composition,
   the content of the substance A being more than 0 mass% and 0.1 mass% or less based on the entire composition,
   the substance A being at least one member selected from the group consisting of an amine, a halogen compound, and toluene, and
   the halogen compound being at least one member selected from the group consisting of chloroform, hexafluorobenzene, 1-chlorobutane, 1,3-bis(trifluoromethyl)benzene, monofluorobenzene, and α,α,α-trifluorotoluene.
Item 13. (Refrigerant 2) A composition comprising 1,1,2-trifluoroethane (HFC-143) and 1,2-dichloro-1,1,2-trifluoroethane (HCFC-123a).
Item 14. (Refrigerant 2) The composition according to Item 13,
   wherein
   the total concentration of the two components comprising HFC-143 and HCFC-123a is 99.5 mass% or more based on the entire composition, and
   the content of HCFC-123a is more than 0 mass% and 0.4 mass% or less based on the entire composition.
Item 15. (Refrigerant 2) The composition according to Item 13,
   wherein
   the composition further comprises 1-chloro-1,1,2-trifluoroethane (HCFC-133b),
   the total concentration of the three components is 99.5 mass% or more based on the entire composition, and
   the content of HCFC-133b is more than 0 mass% and 0.4 mass% or less based on the entire composition.

### Advantageous Effects of Invention

The present disclosure enables efficient purification of HFC-143.

### Brief Description of Drawings

Fig. 1 shows an outline of the process of an embodiment of the present disclosure (Example 1).

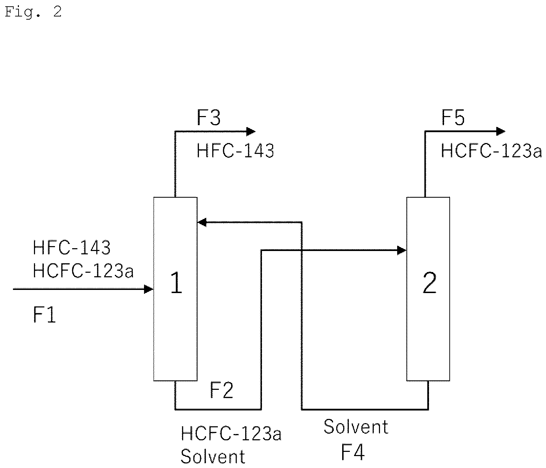

Fig. 2 shows an outline of the process of an embodiment of the present disclosure.
Fig. 3 shows an outline of the process of an embodiment of the present disclosure (Example 11).

### Description of Embodiments

Extensive research was conducted, and it was found that HFC-143 can be efficiently purified by a production method comprising an extractive distillation step of bringing a composition comprising HCFC-133b and HFC-143 into contact with a specific solvent to obtain a composition having a reduced amount of HCFC-133b compared with the original composition.

The present disclosure has been completed as a result of further research based on this finding.

In the present specification, a numerical range expressed using "to" refers to a range that includes the numerical values described before and after "to" as the lower and upper limits (i.e., "or more" and "or less").

In the present specification, an azeotropic composition refers to a composition that behaves as if it were a single substance with no difference in composition between the liquid phase and the gas phase under constant pressure.

In the present specification, an azeotrope-like composition refers to a composition that can form an azeotropic composition, and that has a composition close to an azeotropic composition and behavior like an azeotropic composition. An azeotrope-like composition can be distilled and/or refluxed with little change in composition. Therefore, an azeotrope-like composition can be treated almost the same as an azeotropic composition. One characteristic of azeotrope-like compositions is that the difference in pressure between the boiling point curve and the dew point curve in a pressure-composition diagram is within 5%.

In the present specification, the standard boiling point refers to the boiling point at a standard atmospheric pressure of 1013.25 hPa.

In the present specification, the gauge pressure refers to a relative pressure based on the atmospheric pressure, meaning the pressure difference obtained by subtracting the atmospheric pressure from the absolute pressure. In the present specification, the gauge pressure is denoted with a "G," such as, for example, MPaG. If a "G" is not appended, the pressure is absolute pressure (atmospheric pressure).

In the present specification, the "purity" of a refrigerant refers to the component ratio (mol% or mass%) determined by quantitative analysis using gas chromatography.

In the present specification, the main component refers to a component preferably contained in an amount of 85 mol% to 99.9 mol%, more preferably 90 mol% to 99.9 mol%, even more preferably 95 mol% to 99.9 mol%, and particularly preferably 99 mol% to 99.9 mol%.

In the present specification, extractive distillation refers to a distillation operation to add an extraction solvent to a mixture consisting of two or three components that have very similar standard boiling points and are difficult to separate by ordinary distillation, and having a specific volatility (relative volatility) close to 1, or a combination mixture with an azeotropic composition, to form an extraction mixture, and facilitate separation by adjusting the relative volatility of the original two or three components away from 1. When the relative volatility is 1, separation by distillation is impossible.

In the present specification, specific volatility (α) is defined as follows: if a composition containing at least component A of interest and component B of interest is in vapor-liquid equilibrium, the specific volatility of component A to component B is α_{A→B} = (y_{A}/x_{A}) / (y_{B}/x_{B}) wherein
x_{A} represents the mole fraction of liquid-phase component A,
x_{B} represents the mole fraction of liquid-phase component B,
y_{A} represents the mole fraction of gas-phase component A in equilibrium with liquid-phase component A, and
y_{B} represents the mole fraction of gas-phase component B in equilibrium with liquid-phase component B.

In the present specification, if component A is HFC-143, and component B is HCFC-133b, the specific volatility of component A to component B (i.e., the specific volatility of HFC-143 to HCFC-133b) is denoted as α_{143→133b}.

In the present specification, if component A is HFC-143, and component B is HCFC-123a, the specific volatility of component A to component B (i.e., the specific volatility of HFC-143 to HCFC-123a) is denoted as α₁₄₃→₁₂₃ₐ.

The present disclosure includes the following embodiments.

The production method of the present disclosure is a method for producing purified HFC-143 and includes an extractive distillation step of bringing a composition comprising HCFC-133b and/or HCFC-123a, and HFC-143 into contact with a solvent A to obtain a composition having a reduced amount of HCFC-133b and/or HCFC-123a compared with the original composition.

In particular, it is preferable to apply the production method of the present disclosure when the composition comprising HCFC-133b and/or HCFC-123a, and HFC-143 (also referred to as "extractive distillation composition") is an azeotropic composition or an azeotrope-like composition.

The solvent A is at least one member selected from the group consisting of an amine, a halogen compound, and toluene.

The halogen compound is at least one member selected from the group consisting of chloroform, hexafluorobenzene, 1-chlorobutane, 1,3-bis(trifluoromethyl)benzene, monofluorobenzene, and α,α,α-trifluorotoluene.

### (1) Extractive Distillation Step (1)

The extractive distillation step in the production method of the present disclosure is a step of bringing a composition comprising HCFC-133b and/or HCFC-123a, and HFC-143 (extractive distillation composition) into contact with a solvent A to obtain a composition having a reduced amount of HCFC-133b and/or HCFC-123a compared with the original composition. In other words, the extractive distillation step is a step of subjecting a composition comprising HCFC-133b and/or HCFC-123a, and HFC-143 (extractive distillation composition) to extractive distillation in the presence of a solvent A to obtain a composition having a reduced amount of HCFC-133b and/or HCFC-123a compared with the original composition.

In the present disclosure, the term "reduce" in the extractive distillation step means to reduce the content of a specific compound (HCFC-133b and/or HCFC-123a) in the extractive distillation composition.

The extractive distillation composition contains HCFC-133b and/or HCFC-123a, and HFC-143 in a total concentration of preferably 99.5 mass% or more, more preferably 99.7 mass% or more, even more preferably 99.8 mass% or more, and still even more preferably 99.9 mass% or more.

The extractive distillation composition for use is, for example, a composition comprising HCFC-133b and/or HCFC-123a, and HFC-143 in preferably the total concentrations described above (in particular, an azeotropic composition or azeotrope-like composition). The composition is obtainable in the process of producing HFC-143, readily separable by-products are removed through a pre-separation step (e.g., any distillation step) as necessary.

The extractive distillation composition preferably consists of HFC-143, and HCFC-133b and/or HCFC-123a. The presence of unavoidable impurities is acceptable depending on the conditions for preparing the extractive distillation composition.

HCFC-133b (standard boiling point: 16°C) and/or HCFC-123a (standard boiling point: 28°C), and HFC-143 (standard boiling point: 5°C) are azeotropic or azeotrope-like. The azeotropic composition or azeotrope-like composition of HCFC-133b and/or HCFC-123a, and HFC-143 has a boiling point lower than the boiling point of HCFC-133b and/or HCFC-123a and the boiling point of HFC-143.

The azeotropic composition under 0.1013 MPa is HFC-143/HCFC-133b = 80/20, and the boiling point is 4.4°C.

The azeotropic composition under 0.1013 MPa is HFC-143/HCFC-123a = 93/7, and the boiling point is 4.5°C.

The extractive distillation composition supplied in the extractive distillation step contains HFC-143 in a molar percentage of preferably 80% or more and 99.999% or less, and more preferably 90% or more and 99.999% or less.

The extractive distillation step is preferably a step of bringing the extractive distillation composition into contact with the solvent A to subject the composition to extractive distillation, thereby obtaining a composition that contains HFC-143 and that does not substantially contain HCFC-133b and/or HCFC-123a.

In the present specification, the phrase "not substantially contain HCFC-133b and/or HCFC-123a" means that the content of HCFC-133b and/or HCFC-123a in the composition obtained in the extractive distillation step is preferably less than 1 mass%, more preferably less than 0.5 mass%, and particularly preferably less than 0.1 mass%.

In the extractive distillation step, the extraction solvent for use is at least one solvent A selected from the group consisting of an amine, a halogen compound, and toluene. The halogen compound is at least one member selected from the group consisting of chloroform, hexafluorobenzene, 1-chlorobutane, 1,3-bis(trifluoromethyl)benzene, monofluorobenzene, and α,α,α-trifluorotoluene.

Although the extraction solvent in the present disclosure preferably consists of at least one member selected from the group consisting of an amine, a halogen compound, and toluene, the presence of unavoidable impurities is acceptable to the extent that the impurities do not affect the extractive distillation step. The solvent A may also be simply referred to as "the extraction solvent in the present disclosure."

The amine can be any liquid amine that can be an extraction solvent, and is preferably represented by formula: NR¹R²R³ wherein R¹, R², and R³ are the same or different, and each represents hydrogen or an optionally substituted C₁₋₃ hydrocarbon group, with the proviso that a case in which all of R¹, R², and R³ are hydrogen is excluded.

The amine preferably has a standard boiling point of -10 to 160°C.

The amine is preferably at least one member selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine.

Among these amines, at least one of diethylamine and triethylamine is more preferable because the operation conditions of the distillation column can be easily adjusted to a pressure and temperature at which the operation is easy.

Table 1 below shows the Cas number and the standard boiling point of these amines.

**Table 1**

| Table 1 | | |
|---|---|---|
| Substance | Cas No. | Standard Boiling Point (°C) |
| Monomethylamine | 74-89-5 | -6 |
| Dimethylamine | 124-40-3 | 7.0 |
| Trimethylamine | 75-50-3 | 2.9 |
| Monoethylamine | 75-04-7 | 38 |
| Diethylamine | 109-89-7 | 56 |
| Triethylamine | 121-44-8 | 90 |
| Mono-n-propylamine | 107-10-8 | 48 |
| Di-n-propylamine | 142-84-7 | 110 |
| Tri-n-propylamine | 102-69-2 | 156 |
| Mono-isopropylamine | 75-31-0 | 32 |
| Di-isopropylamine | 108-18-9 | 84 |

These amines may be used alone, or in a combination of two or more.

The halogen compound is a liquid halogen compound that can be an extraction solvent, and is at least one member selected from the group consisting of chloroform, hexafluorobenzene, 1-chlorobutane, 1,3-bis(trifluoromethyl)benzene, monofluorobenzene, and α,α,α-trifluorotoluene.

Among these halogen compounds, at least one member selected from the group consisting of chloroform, hexafluorobenzene, 1-chlorobutane, 1,3-bis(trifluoromethyl)benzene, and monofluorobenzene is particularly preferable from the viewpoint of, for example, ease of availability, ease of handling, and operation temperature.

Table 2 below shows the Cas number and the standard boiling point of these halogen compounds.

**Table 2**

| Table 2 | | |
|---|---|---|
| Substance | Cas No. | Standard Boiling Point (°C) |
| Chloroform | 67-66-3 | 61 |
| Hexafluorobenzene | 392-56-3 | 81 |
| 1-Chlorobutane | 109-69-3 | 78 |
| 1,3-Bis(trifluoromethyl)benzene | 402-31-3 | 116 |
| Monofluorobenzene | 462-06-6 | 85 |
| α,α,α-Trifluorotoluene | 98-08-8 | 102 |

These halogen compounds may be used alone, or in a combination of two or more.

In addition to amines and halogen compounds, toluene can also be used as an extraction solvent in the extractive distillation step.

Table 3 below shows the Cas number and standard boiling point of toluene.

**Table 3**

| Table 3 | | |
|---|---|---|
| Substance | Cas No. | Standard Boiling Point (°C) |
| Toluene | 108-88-3 | 111 |

The mixing ratio of the amine, halogen compound, and toluene in the solvent A is not limited. The solvent A may be only the amine, only the halogen compound, only toluene, or a mixture of two or more of these in any proportions.

Regarding the temperature range of the standard boiling point in the extractive distillation step, the temperature difference may be of a degree that the extraction solvent can be separated from the compound to be separated in the extractive distillation step by simple distillation, stripping, or the like; i.e., the temperature difference is generally 20°C or more. However, if the standard boiling point is too high, the extraction solvent itself may be decomposed.

Therefore, the standard boiling point of the extraction solvent is preferably 30 to 135°C, more preferably 35 to 120°C, even more preferably 40 to 100°C, and particularly preferably 50 to 90°C, from the viewpoint of efficiently performing extractive distillation.

The amount of the extraction solvent used in the extractive distillation step is preferably 1 mol equivalent or more and 30 mol equivalents or less, and more preferably 5 mol equivalents or more and 25 mol equivalents or less, per mol equivalent of the extractive distillation composition supplied to an extractive distillation column.

The concentration of HCFC-133b and/or HCFC-123a in the extractive distillation composition in the extractive distillation step is preferably 30 mol% or less, and more preferably 20 mol% or less. The lower limit of the concentration of HCFC-133b and/or HCFC-123a in the extractive distillation composition in the extractive distillation step is preferably 0.001 mol% or more, more preferably 0.01 mol% or more, and even more preferably 0.1 mol% or more.

The number of theoretical plates of the extractive distillation column used in the extractive distillation step is preferably 10 or more, and more preferably 20 or more. The number of theoretical plates of the extractive distillation column used in the extractive distillation step is preferably 100 or less, and more preferably 70 or less, from an economical viewpoint.

In the extractive distillation step, the extraction solvent is preferably supplied to an upper plate of the extractive distillation column. The extraction solvent used in the extractive distillation step is preferably the extraction solvent that is recovered and recirculated in the extraction solvent recovery step, described later.

In the extractive distillation step, the pressure under which extractive distillation is performed (the pressure of the first distillation column) is preferably 0.05 to 5 MPaG (gauge pressure). The lower limit of the pressure is preferably 0.05 MPaG, more preferably 0.1 MPaG, even more preferably 0.25 MPaG, and particularly preferably 0.5 MPaG. The upper limit of the pressure is preferably 5 MPaG, more preferably 4 MPaG, even more preferably 3 MPaG, and particularly preferably 2 MPaG.

The extractive distillation step can be performed by a discontinuous operation or a continuous operation. The step is preferably performed by a continuous operation from an industrial viewpoint. Further, extractive distillation can be repeated to thereby purify the distillate component to a high purity.

For distilling HFC-143 from the extractive distillation composition in the extractive distillation step, it is preferable to use an extraction solvent that makes the relative volatility (specific volatility) of HFC-143 to HCFC-133b 1.1 or more, and preferably 1.2 or more, upon addition of the extraction solvent.

This increases the gas-phase mole fraction of HFC-143 to thereby increase HFC-143 in the gas phase, thus enabling the separation of HFC-143 from the top of the extractive distillation column; thus, the extraction solvent and HCFC-133b are obtained from the bottom of the extractive distillation column.

For distilling HFC-143 from the extractive distillation composition in the extractive distillation step, it is preferable to use an extraction solvent that makes the relative volatility (specific volatility) of HFC-143 to HCFC-123a 1.1 or more, and preferably 1.2 or more, upon addition of the extraction solvent.

This increases the gas-phase mole fraction of HFC-143 to thereby increase HFC-143 in the gas phase, thus enabling the separation of HFC-143 from the top of the extractive distillation column; thus, the extraction solvent and HCFC-123a are obtained from the bottom of the extractive distillation column.

### (2) Extraction Solvent Recovery Step (3)

The production method of the present disclosure preferably includes an extraction solvent recovery step of recovering the extraction solvent used in the extractive distillation step, and recirculating the recovered extraction solvent to the extractive distillation step.

### (3) Distillation Step (2)

The extraction solvent recovery step includes a distillation step of subjecting the composition comprising HCFC-133b and/or HCFC-123a, and the solvent A obtained from the bottom of the extractive distillation column through the extractive distillation step to distillation to separate the composition into a composition comprising HCFC-133b and/or HCFC-123a as a main component and a composition comprising the extraction solvent as a main component (hereinafter also referred to as "distillation step"), and can be performed by recovering the extraction solvent from the composition comprising the extraction solvent as a main component and recirculating the recovered extraction solvent to the extractive distillation step.

The number of theoretical plates of the solvent recovery column (second distillation column) used in the distillation step is preferably 5 or more, and more preferably 10 or more. The number of theoretical plates of the solvent recovery column is preferably 40 or less, and more preferably 30 or less, from an economical viewpoint.

In the distillation step, the pressure under which distillation is performed is preferably 0.05 to 3 MPaG (gauge pressure). The lower limit of the pressure is preferably 0.05 MPaG, and more preferably 0.1 MPaG. The upper limit of the pressure is preferably 3 MPaG, and more preferably 2.5 MPaG.

The distillation step enables the separation of the composition comprising HCFC-133b and/or HCFC-123a as a main component from the top of the solvent recovery column; thus, the composition comprising the extraction solvent as a main component is obtained from the bottom of the solvent recovery column.

The extraction solvent recovery step can be performed by recovering the extraction solvent from the composition comprising the extraction solvent as a main component obtained from the bottom in the distillation step, and recirculating the extraction solvent to the extractive distillation step. The extraction solvent recovered from the bottom may optionally be further subjected to a separation step, such as rectification, to remove impurities before recirculating it to the extractive distillation step.

The distillation step can be performed by a discontinuous operation or a continuous operation; the step is preferably performed by a continuous operation from an industrial viewpoint.

The production method of the present disclosure preferably includes the extractive distillation step and the extraction solvent recovery step, and more preferably includes an optional pre-distillation step for increasing the purity of an extractive distillation composition, the extractive distillation step, and the extraction solvent recovery step.

### Composition Comprising HFC-143, HCFC-133b and/or HCFC-123a, and Substance A

The present disclosure encompasses a composition comprising 1,1,2-trifluoroethane (HFC-143), 1-chloro-1,1,2-trifluoroethane (HCFC-133b) and/or 1,2-dichloro-1,1,2-trifluoroethane (HCFC-123a), and a substance A (hereinafter also referred to as "refrigerant 1").

The type of substance A is as described regarding the solvent A in the extraction solvent section. That is, the substance A is at least one member selected from the group consisting of an amine, a halogen compound, and toluene.

The halogen compound is at least one member selected from the group consisting of chloroform, hexafluorobenzene, 1-chlorobutane, 1,3-bis(trifluoromethyl)benzene, monofluorobenzene, and α,α,α-trifluorotoluene.

Refrigerant 1 is a composition comprising HFC-143, HCFC-133b and/or HCFC-123a, and a substance A, wherein the total concentration of the three components is 99.5 mass% or more based on the entire composition, the content of HCFC-133b and/or HCFC-123a is more than 0 mass% and 0.4 mass% or less based on the entire composition, and the content of the substance A is more than 0 mass% and 0.1 mass% or less based on the entire composition. The phrase "more than 0 mass%" can also be described as "0.01 mass% or more."

The total concentration of the three components in refrigerant 1 is preferably 99.7 mass% or more, more preferably 99.8 mass% or more, and even more preferably 99.9 mass% or more, based on the entire composition.

It is particularly preferable that refrigerant 1 consists of HFC-143, HCFC-133b and/or HCFC-123a, and the substance A. However, the presence of unavoidable impurities is acceptable.

Although use of refrigerant 1 is not limited, refrigerant 1 can be used in, for example, refrigerants for air conditioning, heat transfer mediums, or refrigerants for car air conditioners.

### Composition Comprising Purified HFC-143

The present disclosure encompasses a composition comprising HFC-143 and a substance A (hereinafter also referred to as "composition 1") as a composition comprising purified HFC-143. The type of substance A is as described above.

Composition 1 is a composition comprising HFC-143 and a substance A, wherein the total concentration of the two components is 99.5 mass% or more based on the entire composition, and the content of the substance A is more than 0 mass% and 0.1 mass% or less based on the entire composition. Composition 1 is a composition comprising a trace amount of extraction solvent (solvent A), among the compositions containing purified HFC-143 ultimately obtained according to the production method of the present disclosure. The phrase "more than 0 mass%" can also be described as "0.01 mass% or more."

The total concentration of the two components in composition 1 is preferably 99.7 mass% or more, more preferably 99.8 mass% or more, and even more preferably 99.9 mass% or more, based on composition 1.

It is particularly preferable that composition 1 consists of HFC-143 and the substance A. However, the presence of unavoidable impurities is acceptable.

Although use of composition 1 is not limited, composition 1 can be used in, for example, leak-detecting agents, stabilizers, or tracers during the use of a refrigerant.

### Composition Comprising HFC-143 and HCFC-123a

The present disclosure encompasses a composition comprising 1,1,2-trifluoroethane (HFC-143) and 1,2-dichloro-1,1,2-trifluoroethane (HCFC-123a) (hereinafter also referred to as "refrigerant 2").

Refrigerant 2 is a composition comprising HFC-143 and HCFC-123a, wherein the total concentration of the two components (HFC-143 and HCFC-123a) is preferably 99.5 mass% or more based on the entire composition, and the content of HCFC-123a is preferably more than 0 mass% and 0.4 mass% or less based on the entire composition. The phrase "more than 0 mass%" can also be described as "0.01 mass% or more."

The total concentration of the two components in refrigerant 2 is more preferably 99.7 mass% or more, even more preferably 99.8 mass% or more, and particularly preferably 99.9 mass% or more, based on the entire composition.

It is particularly preferable that refrigerant 2 consists of HFC-143 and HCFC-123a. However, the presence of unavoidable impurities is acceptable.

Refrigerant 2 may further contain HCFC-133b. The total concentration of the three components is 99.5 mass% or more based on the entire composition, and the content of HCFC-133b is more than 0 mass% and 0.4 mass% or less based on the entire composition. The phrase "more than 0 mass%" can also be described as "0.01 mass% or more."

The total concentration of the three components in refrigerant 2 is preferably 99.7 mass% or more, more preferably 99.8 mass% or more, and even more preferably 99.9 mass% or more, based on the entire composition.

It is particularly preferable that refrigerant 2 consists of HFC-143, HCFC-123a, and HCFC-133b. However, the presence of unavoidable impurities is acceptable.

Although the use of refrigerant 2 is not limited, refrigerant 2 can be used in, for example, refrigerants for air conditioning, heat transfer mediums, or refrigerants for car air conditioners.

### Examples

Examples are provided below to specifically describe the present disclosure. However, the present disclosure is not limited to these Examples.

In the Examples, the concentration of each component, such as HFC-143, was measured with the following measurement device under the following measurement conditions.

### (1) Composition Comprising HFC-143, HCFC-133b, and Substance A

Measurement device: gas chromatography (using an FID detector) Method for calculating the concentration of each component:
- Concentration of HFC-143 = number of moles of HFC-143/(number of moles of HFC-143 + number of moles of HCFC-133b)
- Concentration of HCFC-133b = number of moles of HCFC-133b/(number of moles of HFC-143 + number of moles of HCFC-133b)

### Examples 1 to 9 and Comparative Examples 1 and 2

To a composition containing 80 mol% of HFC-143 and 20 mol% of HCFC-133b, individual solvents were added in an amount of 5-fold mol based on the composition, and each mixture was measured for specific volatility α_{143→133b} of HFC-143 to HCFC-133b at 15°C.

In Example 1, the solvent for use was diethylamine, and the specific volatility α_{143→133b} was 1.8.

In Example 2, the solvent for use was triethylamine, and the specific volatility α_{143→133b} was 2.0.

In Example 3, the solvent for use was 1-chlorobutane, and the specific volatility α_{143→133B} was 1.8.

In Example 4, the solvent for use was 1,3-bis (trifluoromethyl)benzene, and the specific volatility α_{143→133b} was 1.8.

In Example 5, the solvent for use was chloroform, and the specific volatility α_{143→133b} was 1.7.

In Example 6, the solvent for use was hexafluorobenzene, and the specific volatility α_{143→133b} was 1.9.

In Example 7, the solvent for use was monofluorobenzene, and the specific volatility α_{143→133b} was 1.8.

In Example 8, the solvent for use was α,α,α-trifluorotoluene, and the specific volatility α_{143→133b} was 1.9.

In Example 9, the solvent for use was toluene, and the specific volatility α_{143→133b} was 1.7.

In Comparative Example 1, the solvent for use was methanol, and the specific volatility α_{143→133b} was 1.1.

In Comparative Example 2, the solvent for use was ethanol, and the specific volatility α_{143→133b} was 1.2.

Table 4 below summarizes the results.

**Table 4**

| Table 4 | | |
|---|---|---|
| | Solvent | Specific Volatility α_{143→133b} |
| Example 1 | Diethylamine | 1.8 |
| Example 2 | Triethylamine | 2.0 |
| Example 3 | 1-Chlorobutane | 1.8 |
| Example 4 | 1,3-Bis(trifluoromethyl)benzene | 1.8 |
| Example 5 | Chloroform | 1.7 |
| Example 6 | Hexafluorobenzene | 1.9 |
| Example 7 | Monofluorobenzene | 1.8 |
| Example 8 | α,α,α-Trifluorotoluene | 1.9 |
| Example 9 | Toluene | 1.7 |
| Comparative Example 1 | Methanol | 1.1 |
| Comparative Example 2 | Ethanol | 1.2 |

From the results, amines such as diethylamine and triethylamine, halogen compounds such as 1-chlorobutane, 1,3-bis(trifluoromethyl)benzene, chloroform, hexafluorobenzene, monofluorobenzene, and α,α,α-trifluorotoluene, and toluene were found to be effective as an extraction solvent.

### (2) Composition Comprising Purified HFC-143

### Example 10

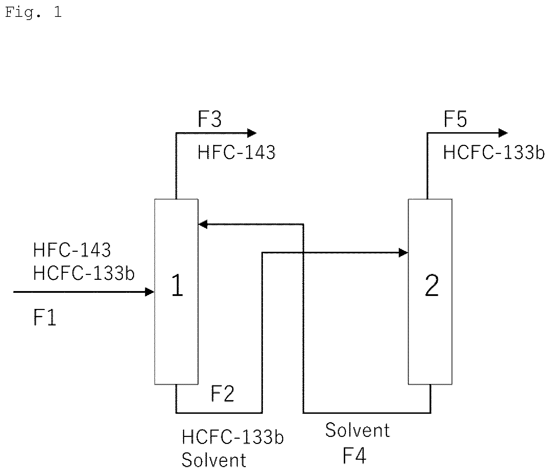

According to the flow diagram shown in Fig. 1, purified HFC-143 was produced by using an extractive distillation composition containing HFC-143 and HCFC-133b as a raw material and hexafluorobenzene as an extraction solvent by a process including the extractive distillation step and the extraction solvent recovery step.

### Extractive Distillation Step

An extractive distillation composition was supplied at a flow rate of 18 mol/hr to an extractive distillation column with 60 theoretical plates from the 30th stage from the top of the extractive distillation column. An extraction solvent (hexafluorobenzene) was supplied from the 3rd stage of the extractive distillation column at a flow rate of 144 mol/hr. The operating pressure of the extractive distillation column was 0.3 MPaG, and the column top temperature was 42°C.

### Extraction Solvent Recovery Step

The bottom product extracted from the column bottom in the extractive distillation step was fed from the 14th stage from the top of an extraction solvent recovery column with 20 theoretical plates (hereinafter referred to as "solvent recovery column"). HCFC-133b was recovered from the top of the column with a purity of 99.99%. The operating pressure of the solvent recovery column was 0.2 MPaG, and the column top temperature was 48°C.

The bottom product containing hexafluorobenzene extracted from the bottom of the recovery column (flow rate of hexafluorobenzene: 144 mol/hr) was recirculated to the extractive distillation step and used.

### Material Balance of Example 10 according to Process in Fig. 1

In F1, HFC-143 was 14.4 mol/hr, HCFC-133b was 3.6 mol/hr, and hexafluorobenzene was 0 mol/hr.

In F2, HFC-143 was 0.01 mol/hr, HCFC-133b was 3.7 mol/hr, and hexafluorobenzene was 144 mol/hr.

In F3, HFC-143 was 14.39 mol/hr, HCFC-133b was 0.0001 mol/hr, and hexafluorobenzene was present in a trace amount (0.1 ppm or less).

In F4, HFC-143 was present in a trace amount (0.1 ppm or less), HCFC-133b was 0.1 mol/hr, and hexafluorobenzene was 144 mol/hr.

In F5, HFC-143 was 0.01 mol/hr, HCFC-133b was 3.6 mol/hr, and hexafluorobenzene was 0 mol/hr.

Table 5 below summarizes the material balance of Example 10.

**Table 5**

| Table 5 | | | | | |
|---|---|---|---|---|---|
| | F1 | F2 | F3 | F4 | F5 |
| HFC-143 | 14.4 | 0.01 | 14.39 | 0.1 ppm or less | 0.01 |
| HCFC-133b | 3.6 | 3.7 | 0.0001 | 0.1 | 3.6 |
| Hexafluorobenzene | 0 | 144 | 0.1 ppm or less | 144 | 0 |
| Unit: mol/hr | | | | | |

The amines shown in Examples 1 and 2, the halogen compounds shown in Examples 3 to 8, and toluene shown in Example 9 all had a specific volatility α_{143→133b} of HFC-143 to HCFC-133b of 1.7 to 2.0, which was sufficiently larger than that of any Comparative Example (1.2 or less), meaning a higher HFC-143 concentration in a gas phase by 50% or more; this indicates notably improved separation efficiency.

Therefore, the process of the present disclosure using at least one extraction solvent (solvent A) selected from the group consisting of an amine, a halogen compound, and toluene in the extractive distillation step is very effective in the separation of HCFC-133b and HFC-143.

### Example 11

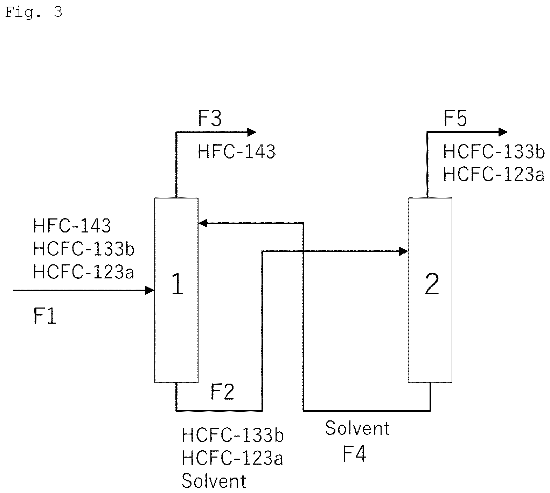

According to the flow diagram shown in Fig. 3, purified HFC-143 was produced by using an extractive distillation composition containing HFC-143, HCFC-133b, and HCFC-123a as a raw material and hexafluorobenzene as an extraction solvent by a process including the extractive distillation step and the extraction solvent recovery step.

### Extractive Distillation Step

An extractive distillation composition was supplied at a flow rate of 18 mol/hr to an extractive distillation column with 60 theoretical plates from the 30th stage from the top of the extractive distillation column. An extraction solvent (hexafluorobenzene) was supplied from the 3rd stage of the extractive distillation column at a flow rate of 178 mol/hr. The operating pressure of the extractive distillation column was 0.3 MPaG, and the column top temperature was 42°C.

### Extraction Solvent Recovery Step

The bottom product extracted from the column bottom in the extractive distillation step was fed from the 14th stage from the top of an extraction solvent recovery column with 20 theoretical plates (hereinafter referred to as "solvent recovery column"). HCFC-133b was recovered from the top of the column with a purity of 99.99%. The operating pressure of the solvent recovery column was 0.2 MPaG, and the column top temperature was 50°C.

The bottom product containing hexafluorobenzene extracted from the bottom of the recovery column (flow rate of hexafluorobenzene: 178 mol/hr) was recirculated to the extractive distillation step and used.

### Material Balance of Example 11 according to Process in Fig. 3

In F1, HFC-143 was 14.4 mol/hr, HCFC-133b was 3.6 mol/hr, HCFC-123a was 0.6 mol/hr, and hexafluorobenzene was 0 mol/hr.

In F2, HFC-143 was 0.01 mol/hr, HCFC-133b was 3.7 mol/hr, HCFC-123a was 0.61 mol/hr, and hexafluorobenzene was 144 mol/hr.

In F3, HFC-143 was 14.39 mol/hr, HCFC-133b was 0.0001 mol/hr, HCFC-123a was undetected (0.0001 mol/hr or less), and hexafluorobenzene was present in a trace amount (0.1 ppm or less).

In F4, HFC-143 was present in a trace amount (0.1 ppm or less), HCFC-133b was 0.1 mol/hr, HCFC-123a was 0.01 mol/hr, and hexafluorobenzene was 144 mol/hr.

In F5, HFC-143 was 0.01 mol/hr, HCFC-133b was 3.6 mol/hr, HCFC-123a was 0.6 mol/hr, and hexafluorobenzene was 0 mol/hr.

Table 6 below summarizes the material balance of Example 11.

**Table 6**

| Table 6 | | | | | |
|---|---|---|---|---|---|
| | F1 | F2 | F3 | F4 | F5 |
| HFC-143 | 14.4 | 0.01 | 14.39 | 0.1 ppm or less | 0.01 |
| HCFC-133b | 3.6 | 3.7 | 0.0001 | 0.1 | 3.6 |
| HCFC-123a | 0.6 | 0.61 | 0 | 0.01 | 0.6 |
| Hexafluorobenzene | 0 | 144 | 0.1 ppm or less | 144 | 0 |
| Unit: mol/hr | | | | | |

### Example 12

Table 7 shows vapor-liquid equilibrium data for azeotropic and azeotrope-like compositions of HFC-143 and HCFC-123a.

**Table 7**

| Table 7 (HFC-143, HCFC-123a) | | | | | | | | |
|---|---|---|---|---|---|---|---|---|
| Temperature (°C) | Pressure (MPa abs) | Liquid Phase (HFC-143) | Gas Phase (HFC-143) | | Temperature (°C) | Pressure (MPa abs) | Liquid Phase (HFC-143) | Gas Phase (HFC-143) |
| 20 | 0.076 | 0.000 | 0.000 | | 40 | 0.349 | 0.600 | 0.783 |
| 20 | 0.099 | 0.100 | 0.309 | | 40 | 0.362 | 0.700 | 0.822 |
| 20 | 0.120 | 0.200 | 0.487 | | 40 | 0.372 | 0.800 | 0.860 |
| 20 | 0.138 | 0.300 | 0.602 | | 40 | 0.377 | 0.900 | 0.910 |
| 20 | 0.153 | 0.400 | 0.681 | | 40 | 0.378 | 0.932 | 0.932 |
| 20 | 0.165 | 0.500 | 0.740 | | 40 | 0.374 | 1.000 | 1.000 |
| 20 | 0.174 | 0.600 | 0.785 | | 60 | 0.277 | 0.000 | 0.000 |
| 20 | 0.181 | 0.700 | 0.824 | | 60 | 0.366 | 0.100 | 0.314 |
| 20 | 0.185 | 0.800 | 0.862 | | 60 | 0.443 | 0.200 | 0.489 |
| 20 | 0.188 | 0.900 | 0.910 | | 60 | 0.508 | 0.300 | 0.600 |
| 20 | 0.189 | 0.930 | 0.930 | | 60 | 0.561 | 0.400 | 0.677 |
| 20 | 0.187 | 1.000 | 1.000 | | 60 | 0.603 | 0.500 | 0.734 |
| 40 | 0.152 | 0.000 | 0.000 | | 60 | 0.635 | 0.600 | 0.779 |
| 40 | 0.200 | 0.100 | 0.312 | | 60 | 0.659 | 0.700 | 0.819 |
| 40 | 0.242 | 0.200 | 0.489 | | 60 | 0.676 | 0.800 | 0.858 |
| 40 | 0.278 | 0.300 | 0.602 | | 60 | 0.686 | 0.900 | 0.909 |
| 40 | 0.307 | 0.400 | 0.680 | | 60 | 0.687 | 0.933 | 0.933 |
| 40 | 0.331 | 0.500 | 0.738 | | 60 | 0.681 | 1.000 | 1.000 |

### Example 13

Table 8 shows vapor-liquid equilibrium data for azeotropic and azeotrope-like compositions of HFC-143 and HCFC-133b.

**Table 8**

| Table 8 (HFC-143, HCFC-133b) | | | | | | | | |
|---|---|---|---|---|---|---|---|---|
| Temperature (°C) | Pressure (MPa abs) | Liquid Phase (HFC-143) | Gas Phase (HFC-143) | | Temperature (°C) | Pressure (MPa abs) | Liquid Phase (HFC-143) | Gas Phase (HFC-143) |
| 20 | 0.116 | 0.000 | 0.000 | | 40 | 0.362 | 0.600 | 0.666 |
| 20 | 0.144 | 0.100 | 0.266 | | 40 | 0.368 | 0.700 | 0.737 |
| 20 | 0.159 | 0.200 | 0.383 | | 40 | 0.372 | 0.800 | 0.815 |
| 20 | 0.168 | 0.300 | 0.461 | | 40 | 0.374 | 0.900 | 0.903 |
| 20 | 0.175 | 0.400 | 0.525 | | 40 | 0.374 | 0.945 | 0.945 |
| 20 | 0.180 | 0.500 | 0.588 | | 40 | 0.374 | 1.000 | 1.000 |
| 20 | 0.183 | 0.600 | 0.655 | | 60 | 0.402 | 0.000 | 0.000 |
| 20 | 0.186 | 0.700 | 0.727 | | 60 | 0.493 | 0.100 | 0.255 |
| 20 | 0.187 | 0.800 | 0.808 | | 60 | 0.547 | 0.200 | 0.383 |
| 20 | 0.187 | 0.873 | 0.873 | | 60 | 0.584 | 0.300 | 0.470 |
| 20 | 0.187 | 0.900 | 0.898 | | 60 | 0.611 | 0.400 | 0.541 |
| 20 | 0.187 | 1.000 | 1.000 | | 60 | 0.632 | 0.500 | 0.607 |
| 40 | 0.226 | 0.000 | 0.000 | | 60 | 0.649 | 0.600 | 0.674 |
| 40 | 0.279 | 0.100 | 0.261 | | 60 | 0.662 | 0.700 | 0.744 |
| 40 | 0.309 | 0.200 | 0.385 | | 60 | 0.672 | 0.800 | 0.821 |
| 40 | 0.328 | 0.300 | 0.467 | | 60 | 0.678 | 0.900 | 0.906 |
| 40 | 0.342 | 0.400 | 0.535 | | 60 | 0.680 | 0.977 | 0.977 |
| 40 | 0.353 | 0.500 | 0.599 | | 60 | 0.681 | 1.000 | 1.000 |

### Description of the Reference Numerals

1. Extractive distillation column
2. Solvent recovery column

## Claims

1. A method for producing purified 1,1,2-trifluoroethane (HFC-143), comprising (1) an extractive distillation step of bringing a composition comprising 1-chloro-1,1,2-trifluoroethane (HCFC-133b) and/or 1,2-dichloro-1,1,2-trifluoroethane (HCFC-123a), and HFC-143 into contact with a solvent A to obtain a composition having a reduced amount of HCFC-133b and/or HCFC-123a compared with the original composition,
the solvent A being at least one member selected from the group consisting of an amine, a halogen compound, and toluene, and
the halogen compound being at least one member selected from the group consisting of chloroform, hexafluorobenzene, 1-chlorobutane, 1,3-bis(trifluoromethyl)benzene, monofluorobenzene, and α,α,α-trifluorotoluene.

2. The method according to claim 1, further comprising (2) a distillation step of subjecting a composition comprising HCFC-133b and/or HCFC-123a, and the solvent A obtained from the bottom of an extractive distillation column through the extractive distillation step (1) to distillation to separate the composition into HCFC-133b and/or HCFC-123a, and the solvent A.

3. The method according to claim 1, wherein the extractive distillation step (1) comprises extractive distillation performed under a pressure of 0.05 to 5 MPaG (gauge pressure) using a first distillation column.

4. The method according to claim 2, wherein the distillation step (2) comprises distillation performed under a pressure of 0.05 to 3 MPaG (gauge pressure) using a second distillation column.

5. The method according to claim 1, further comprising (3) a solvent recovery step of recovering the solvent A used in the extractive distillation step (1), and recirculating the recovered solvent A to the extractive distillation step (1).

6. The method according to claim 1, wherein the amine is represented by formula: NR¹R²R³
wherein R¹, R², and R³ are the same or different, and each represents hydrogen or an optionally substituted C₁₋₃ hydrocarbon group, with the proviso that a case in which all of R¹, R², and R³ are hydrogen is excluded.

7. The method according to claim 1, wherein the amine has a standard boiling point of -10 to 160°C.

8. The method according to claim 1, wherein the amine is at least one member selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine.

9. The method according to claim 1, wherein the amine is at least one member selected from the group consisting of diethylamine and triethylamine.

10. The method according to claim 1, wherein the halogen compound is at least one member selected from the group consisting of chloroform, hexafluorobenzene, 1-chlorobutane, 1,3-bis(trifluoromethyl)benzene, and monofluorobenzene.

11. A composition comprising 1,1,2-trifluoroethane (HFC-143), 1-chloro-1,1,2-trifluoroethane (HCFC-133b) and/or 1,2-dichloro-1,1,2-trifluoroethane (HCFC-123a), and a substance A,
the total concentration of the three components being 99.5 mass% or more based on the entire composition,
the content of HCFC-133b and/or HCFC-123a being more than 0 mass% and 0.4 mass% or less based on the entire composition,
the content of the substance A being more than 0 mass% and 0.1 mass% or less based on the entire composition,
the substance A being at least one member selected from the group consisting of an amine, a halogen compound, and toluene, and
the halogen compound being at least one member selected from the group consisting of chloroform, hexafluorobenzene, 1-chlorobutane, 1,3-bis(trifluoromethyl)benzene, monofluorobenzene, and α,α,α-trifluorotoluene.

12. A composition comprising 1,1,2-trifluoroethane (HFC-143) and a substance A,
the total concentration of the two components being 99.5 mass% or more based on the entire composition,
the content of the substance A being more than 0 mass% and 0.1 mass% or less based on the entire composition,
the substance A being at least one member selected from the group consisting of an amine, a halogen compound, and toluene, and
the halogen compound being at least one member selected from the group consisting of chloroform, hexafluorobenzene, 1-chlorobutane, 1,3-bis(trifluoromethyl)benzene, monofluorobenzene, and α,α,α-trifluorotoluene.

13. A composition comprising 1,1,2-trifluoroethane (HFC-143) and 1,2-dichloro-1,1,2-trifluoroethane (HCFC-123a).

14. The composition according to claim 13,
wherein
the total concentration of the two components comprising HFC-143 and HCFC-123a is 99.5 mass% or more based on the entire composition, and
the content of HCFC-123a is more than 0 mass% and 0.4 mass% or less based on the entire composition.

15. The composition according to claim 13,
wherein
the composition further comprises 1-chloro-1,1,2-trifluoroethane (HCFC-133b),
the total concentration of the three components is 99.5 mass% or more based on the entire composition, and
the content of HCFC-133b is more than 0 mass% and 0.4 mass% or less based on the entire composition.
